# EUROPÄISCHE PATENTSCHRIFT

(11) **EP 1 189 281 B1**
(45) Veröffentlichungstag und Bekanntmachung des Hinweises auf die Patenterteilung: **22.08.2007**
(21) Anmeldenummer: 01126916.4
(22) Anmeldetag: 23.06.1999
(51) Int. Cl.: H01L 25/065, H01L 25/07, G01N 27/403, G01N 27/414, G01N 27/28, G01N 33/487, A61B 5/00, A61M 37/00

(54) **Chip-Anordnung**
Chip-arrangement
Agencement de puce électronique

(30) Priorität: 30.06.1998 DE 19829121
(43) Veröffentlichungstag der Anmeldung: 20.03.2002
(62) Teilanmeldung aus: 99112071.8
(73) Patentinhaber: Micronas GmbH, 79008 Freiburg (DE)
(72) Erfinder: Igel, Günter Dipl.-Ing., 79331 Teningen (DE); Lehmann, Mirko Dipl.-Phys., 79102 Freiburg (DE); Sieben, Ulrich Dr., 79276 Reute (DE); Gahle, Hans-Jürgen Dr., 79312 Emmendingen (DE); Baumann, Werner Dr., 77815 Bühl (DE); Ehret, Ralf Dr., 18057 Rostock (DE)
(74) Vertreter: Huwer, Andreas

(56) Entgegenhaltungen:
- EP-A- 0 190 005
- EP-A- 0 399 227
- WO-A-87/05747
- WO-A-97/42478
- WO-A-98/00193
- DE-A1- 2 736 200
- US-A- 5 114 859

## Beschreibung

Die Erfindung betrifft eine Chip-Anordnung mit einer Substratplatte, die wenigstens einen Durchbruch aufweist, in den ein Trägerchip eingesetzt oder einsetzbar ist, der an einer Trägerchip-Oberfläche wenigstens eine integrierte Leiterbahn aufweist, die wenigstens ein elektrisches oder elektronisches Bauelement, insbesondere einen Sensor, mit zumindest einem elektrischen Anschlußkontakt verbindet, wobei der Trägerchip derart in den Durchbruch eingesetzt oder einsetzbar ist, daß er mit seinen Enden die einander abgewandten flachseitigen Oberflächen der Substratplatte überragt und dadurch Überstände bildet, wobei an dem die eine Oberfläche überragenden Überstand das Bauelement und an dem die andere Oberfläche überragenden Überstand der Anschlußkontakt angeordnet ist, wobei die das Bauelement und den Anschlußkontakt miteinander verbindende Leiterbahn den Durchbruch der Substratplatte durchsetzt, und wobei zwischen der Substratplatte und dem Trägerchip eine Abdichtung vorgesehen ist.

Aus EP 0 399 227 A1 kennt man bereits eine derartige Chip-Anordnung, die zur Untersuchung von Flüssigkeiten, wie z.B. von Blut vorgesehen ist. Die Anordnung weist ein Gehäuse auf, das eine Meßkammer umgrenzt, die von der zu untersuchenden Flüssigkeit durchströmt wird. In einer Gehäusewand weist das Gehäuse einen Durchbruch auf, in den ein Trägerchip eingesetzt ist, der drei als Mikrosensoren ausgebildete Bauelemente aufweist, die über Leiterbahnen mit Anschlußkontakten des Trägerchips verbunden sind. Der Trägerchip ist so in den Durchbruch eingesetzt, daß er mit seinen Enden die einander abgewandten Seiten der Gehäusewand überragt und dadurch Überstände bildet. An dem in der Meßkammer liegenden Ende sind die Bauelemente und an dem die äußere Oberfläche des Gehäuses überragenden Überstand die Anschlußkontakte angeordnet. Dabei durchsetzen die die Bauelemente mit den Anschlußkontakten miteinander verbindenden Leiterbahnen den Durchbruch der Gehäusewand. Die Chip-Anordnung ermöglicht zwar die Untersuchung von Flüssigkeiten, eignet sich jedoch nicht für eine Untersuchung oder Behandlung des definierten Bereiches im Inneren eines weichen Körpers.

Es besteht deshalb die Aufgabe, eine Chip-Anordnung der eingangs genannten Art zu schaffen, die es auf einfache Weise ermöglicht, einen definierten Bereich im Inneren eines weichen Körpers zu untersuchen oder zu behandeln.

Die Lösung dieser Aufgabe besteht bei einer Anordnung der eingangs genannten Art darin, daß sich der Querschnitt des das elektrische oder elektronische Bauelement aufweisenden Überstandes ausgehend von der Oberfläche der Substratplatte zu der am weitesten vorstehenden Stelle des Überstandes verjüngt.

Der das Bauelement aufweisende Überstand weist also eine Spitze auf und kann dadurch in einen zu untersuchenden oder zu behandelnden weichen Körper eingesteckt werden, wobei die flachseitige Oberfläche der Substratplatte als Anschlagfläche für den Körper dient, welche die Einbringtiefe des spitzen Überstands in dem Körper begrenzt. Dadurch ist es möglich, die Bauelemente auf einfache Weise in einer definierten, durch das Überstandmaß des Überstands vorgegebenen Tiefe in dem zu untersuchenden Körper oder Medium zu positionieren. Bei einer Chip-Anordnung, bei der das elektronische Bauelement ein Sensor ist, kann die Substratplatte mit ihrer Flachseite beispielsweise auf eine zu untersuchende Hautschicht aufgelegt werden, wobei der den Sensor aufweisende spitze Überstand mit einer der Höhe des Überstands entsprechenden definierten Tiefe in die Hautschicht eindringt, so daß dort Meßwerte entnommen werden können. So können zum Beispiel die Glucose-Konzentration, die Feuchtigkeit der Haut, eine lonenkonzentration, ein Gasgehalt oder dergleichen physiologische Parameter gemessen werden, die Aussagen über die Vitalität der Haut und/oder des dahinter befindlichen Gewebebereiches ermöglichen. Dabei ist es sogar möglich, daß die an der Hautschicht anliegende Substratplatte parallel zur Oberfläche der Hautschicht verschoben wird, so daß der den Sensor aufweisende Überstand parallel zur Oberfläche der Hautschicht durch diese hindurch gezogen wird. Dadurch kann auf einfache Weise entlang einer parallel zur Oberfläche der Hautschicht verlaufenden Linie ein Meßprofil erstellt werden.

Das elektrische oder elektronische Bauelement ist an dem an der einen Substratplatten-Oberfläche befindlichen Überstand und der damit über die in dem Trägerchip integrierte Leiterbahn verbundene Anschlußkontakt an dem an der anderen Substratplatten-Oberfläche befindlichen Überstand angeordnet. Der Anschlußkontakt befindet sich also an der dem elektrischen oder elektronischen Bauelement abgewandten Rückseite der Substratplatte, so daß die im Bereich des das Bauelement aufweisenden Überstands befindlichen Leiterbahnbereiche vollständig mit einer Passivierungsschicht abgedeckt werden können. Eine solche Passivierungsschicht kann beispielsweise in Dünnschichttechnologie mit großer Genauigkeit und Feuchtigkeitsfestigkeit hergestellt werden, so daß eine Korrosion an der in den Trägerchip integrierten Leiterbahn durch das mit dem elektrischen oder elektronischen Bauelement zu untersuchende oder zu behandelnde Medium weitestgehend vermieden wird. Die zwischen dem Trägerchip und der Substratplatte angeordnete Abdichtung verhindert, daß das an der Vorderseite der Substratplatte befindliche Medium zu dem an der Rückseite der Substratplatte angeordneten Anschlußkontakt gelangen kann. Der in der Substratplatte angeordnete Durchbruch kann beispielsweise mittels Ultraschallbohren in die Substratplatte eingebracht werden. Die Chip-Anordnung ist somit einfach und kostengünstig herstellbar. Da eine Kunststoffmasse zum Eingießen von Bondpads entfallen kann, weist die Chip-Anordnung außerdem besonders kompakte Abmessungen auf.

Zweckmäßigerweise ist der Trägerchip lösbar mit der Substratplatte verbindbar. Der Trägerchip kann dann gegebenenfalls leicht ausgetauscht werden, wenn das Bauelement seine vorgesehene Lebensdauer erreicht hat oder wenn es durch einen Kontakt mit einem zu untersuchenden oder zu behandelnden, chemisch agressiven Medium, einmal ausfallen sollte.

Bei einer bevorzugten und besonders vorteilhaften Ausführungsform der Erfindung ist der Trägerchip mit einem den Durchbruch begrenzenden Wandungsbereich der Substratplatte verklebt. Der zwischen dem Trägerchip und der Substratplatte angeordnete Klebstoff dient dann einerseits dazu, den Trägerchip an der Substratplatte zu fixieren und dichtet andererseits aber auch den Durchbruch der Substratplatte gegen den Trägerchip ab, so daß ein an der Vorderseite der Substratplatte im Bereich des elektrischen oder elektronischen Bauelements befindliches Medium nicht an die den Anschlußkontakt aufweisende Rückseite der Substratplatte gelangen kann. Der Klebstoff gleicht außerdem Toleranzen in den Abmessungen des Trägerchips und/oder dem in der Substratplatte befindlichen Wandungsdurchbruch, in den der Trägerchip eingesetzt ist, aus. Die Chip-Anordnung ist dadurch noch einfacher und kostengünstiger herstellbar.

Zweckmäßigerweise ist der Trägerchip mit seiner Erstreckungsebene rechtwinklig zu einer flachseitigen Oberfläche der Substratplatte angeordnet. Die den Durchbruch begrenzenden Seitenflächen der Substratplatte können dann rechtwinklig zu deren flachseitiger Oberfläche angeordnet sein, was das Einbringen des Durchbruchs in die Substratplatte erleichtert.

Eine besonders vorteilhafte Ausführungsform der Erfindung sieht vor, daß die das elektrische oder elektronische Bauelement aufweisende Trägerchip-Oberfläche schräg zur flachseitigen Oberfläche der Substratplatte angeordnet ist und mit dieser vorzugsweise einen spitzen Winkel einschließt. Das elektrische oder elektronische Bauelement ist dann in einem durch den Trägerchip und die Substratplatte begrenzten Eckbereich angeordnet, so daß nur Partikel, die eine durch die Abmessungen des Eckbereichs vorgegebene Größe nicht überschreiten, mit dem elektrischen oder elektronischen Bauelement in Kontakt geraten können. Somit ergibt sich ein einfach aufgebauter mechanischer Filter, der das Vordringen größerer Partikel zu dem Bauelement verhindert.

Eine vorteilhafte Ausführungsform der Erfindung sieht vor, daß der Trägerchip bei der Montage der Chip-Anordnung in wenigstens zwei unterschiedlichen Lagen in den Durchbruch der Substratplatte einsetzbar ist, daß in einer dieser Lagen wenigstens ein elektrisches oder elektronisches Bauelement an einem eine flachseitige Oberfläche der Substratplatte überragenden Überstand des Trägerchips und der (die) diesem (diesen) Bauelement(en) zugeordnete(n) Anschlußkontakt(e) an dem die andere flachseitige Oberfläche der Substratplatte überragenden Überstand angeordnet ist, und daß in der anderen Lage des Trägerchips das (die) Bauelement(e) und der (die) Anschlußkontakt(e) an demselben, eine flachseitige Oberfläche der Substratplatte überragenden Überstand des Trägerchips angeordnet sind. Dadurch ist es möglich, das Baulelement durch entsprechendes Einsetzen des Trägerchips in die Substratplatte nur für die Dauer einer Messung oder einer Behandlung mit einem an einer flachseitigen Oberflächen der Substratplatte befindlichen Objekt, beispielsweise einem chemisch agressiven Medium, in Berührung zu bringen, während das Baulelement außerhalb der Meß- oder Behandlungsphase an der dem Objekt abgewandten flachseitigen Oberflächen der Substratplatte angeordnet ist. Das Bauelement kommt also nur vorübergehend mit dem agressiven Medium in Verbindung, wodurch sich seine Lebensdauer entsprechend verlängert.

Bei einer besonders vorteilhaften Weiterbildung der Erfindung ist vorgesehen, daß der Trägerchip bei der Montage der Chip-Anordnung in wenigstens zwei unterschiedlichen Lagen in den Durchbruch der Substratplatte einsetzbar ist, daß der Trägerchip wenigstens zwei elektrische oder elektronische Bauelemente aufweist, die jeweils mittels wenigstens einer Leiterbahn mit zumindest einem ihnen jeweils zugeordneten elektrischen Anschlußkontakt verbunden sind, und daß je nach gewählter Lage des Trägerchips jeweils wenigstens eines dieser Bauelemente an einem eine flachseitige Oberfläche der Substratplatte überragenden Überstand des Trägerchips und der (die) diesem (diesen) Bauelement(en) zugeordnete(n) Anschlußkontakt an dem die andere flachseitige Oberfläche der Substratplatte überragenden Überstand angeordnet ist. Dadurch ist je nach gewählter Lage des Trägerchips ein anderes Bauelement oder sogar mehrere andere Bauelemente an dem in Gebrauchsstellung dem zu untersuchenden oder zu behandelnden Objekt zugewandten Überstand des Trägerchips angeordnet. Bei einem Trägerchip mit mehreren gleichen Bauelementen verlängert sich dadurch die Lebensdauer der Chip-Anordnung entsprechend, da ein Bauelement, das beispielsweise durch einen längeren Kontakt mit einem chemisch agressiven Medium unbrauchbar geworden ist, durch entsprechendes Umsetzen des Trägerchips auf einfache Weise durch ein anderes, funktionsfähiges Bauelement ersetzt werden kann. Der Trägerchip kann aber auch voneinander verschiedene Bauelemente aufweisen. Dadurch ergibt sich ein Bausatz zum Erstellen einer Chip-Anordnung, mit dem je nach gewählter Lage des Trägerchips in dem Durchbruch der Substratplatte unterschiedliche Chip-Anordnungen hergestellt werden können. Die elektrischen oder elektronischen Bauelemente können beispielsweise am Umfang des Trägerchips verteilt in dessen flachseitige Oberfläche integriert sein, wobei der Trägerchip in unterschiedlichen Drehlagen in Bezug zu der Normalen auf diese Oberfläche in die Substratplatte einsetzbar ist. Abhängig von der jeweiligen Drehlage des Trägerchips sind dann jeweils andere Bauelemente oder Sensoren an der Vorderseite der Substratplatte angeordnet, während die diesen zugeordneten Anschlußkontakte sich jeweils an der Rückseite der Substratplatte befinden.

Vorteilhaft ist, wenn auf dem elektrischen oder elektronischen Bauelement eine ionendurchlässige Membran angeordnet ist. Dadurch können Ionen bis an das Bauelement beziehungsweise den Sensor gelangen, während andere Substanzen durch die Membran von dem Sensor ferngehalten werden. Dabei ist es sogar möglich, daß die Membran nur für bestimmte Ionen durchlässig ist, so daß deren Konzentration in einem zu untersuchenden Medium selektiv gemessen werden kann. Zweckmäßigerweise wird die Membran nach dem Einsetzen des Trägerchips in die Substratplatte auf das elektrische oder elektronische Bauelement aufgetragen. Dazu wird das Membranmaterial zunächst in einer flüchtigen Flüssigkeit, beispielsweise in Alkohol oder Aceton gelöst. Die Chip-Anordnung wird so ausgerichtet, daß die das Bauelement aufweisende Oberfläche des Trägerchips schräg zur Horizontalen, insbesondere vertikal verläuft. Dann wird auf die benachbart zu dem Bauelement angeordnete, quer zu der das Bauelement aufweisenden Oberfläche des Trägerchips verlaufende stirnseitige Randfläche des Trägerchips eine geringe Menge der das Membranmaterial enthaltenden Flüssigkeit aufgetragen, derart, daß ein Teil dieser Flüssigkeit schwerkraftbedingt von der stirnseitigen Randfläche des Trägerchips über das an der quer dazu angeordneten Oberfläche des Trägerchips befindliche elektrische oder elektronische Bauelement fließt, so daß sich auf diesem eine dünne Flüssigkeitsschicht bildet. Nach dem Verdunsten dieser Flüssigkeitsschicht verbleibt dann auf dem elektrischen oder elektronischen Bauelement eine Membranschicht, die auf dem Bauelement eine gleichmäßige Dicke aufweist.

Bei einer vorteilhaften Ausführungsform der Erfindung weist die Substratplatte im Bereich des Meß- oder Wirkraumes des elektrischen oder elektronischen Bauelements wenigstens einen Vorsprung auf, der zusammen mit dem das Bauelement aufweisenden Überstand einen mechanischen Filter bildet. Dabei wird unter einem Wirkraum bei einem eine elektromagnetische Strahlung aussendenden Bauelement der Raum verstanden, in den das Bauelement die Strahlung aussendet. Entsprechend wird bei einem Bauelement, von dem ein elektrisches und/oder magnetisches Feld ausgeht, der Raum verstanden, in dem dieses Feld wirksam ist. Der mechanische Filter weist also einen mit dem das Bauelement aufweisenden Überstand zusammenwirkenden Vorsprung auf, wobei zwischen dem Bauelement und dem Vorsprung ein Freiraum angeordnet ist, der den Zugang zu dem Bauelement bildet. Dadurch werden Partikel, deren Abmessungen größer sind als diejenigen des Freiraums von dem Meß- oder Wirkraum des Bauelements ferngehalten, während kleinere Partikel in den Meß- oder Wirkraum und gegebenenfalls bis an das Bauelement selbst gelangen können. Der Vorsprung kann auch ein an der Substratplatte befindlicher Absatz oder eine Stufe sein.

Eine Ausführungsform sieht vor, daß der Vorsprung des mechanischen Filters durch den Überstand eines in einen Durchbruch der Substratplatte eingesetzten Plättchens gebildet ist. Der den Vorsprung kann dann bei der Herstellung der Chip-Anordnung in gleicher Weise an der Substratplatte angebracht werden, wie der das elektrische oder elektronische Bauelement aufweisende Überstand des Trägerchips. Die Chip-Anordnung ist dadurch noch einfacher herstellbar. Gegebenenfalls kann der Vorsprung des mechanischen Filters auch durch den Überstand eines weiteren Trägerchips gebildet sein.

Zum Filtern kleiner Partikel, beispielsweise solcher mit einem Durchmesser, der kleiner als 1 µm ist, ist es vorteilhaft, wenn an dem Trägerchip ein Körper anliegt, der das elektrische oder elektronische Bauelement überdeckt, daß als Abstandshalter an dem Trägerchip mindestens ein seitlich über die Oberflächenebene des Bauelements vorstehender, an dem Körper anliegender Bereich und/oder an dem Körper ein seitlich über den das Bauelement überdeckenden Oberflächenbereich vorstehender, an dem Trägerchip anliegender Bereich angeordnet ist, derart, daß zwischen dem Bauelement und dem Körper ein den Zugang zu dem Bauelement bildender Freiraum oder Spalt angeordnet ist. Dadurch ist der Körper bei der Montage der Chip-Anordnung einfacher und mit größerer Genauigkeit an dem Trägerchip positionierbar. Der gegenüber dem Bauelement vorstehende Bereich kann mit bekannten Verfahren der Halbleitertechnik, beispielsweis in Maskentechnik mit großer Maßgenauigkeit hergestellt werden, was insbesondere die Realisierung kleiner Spaltmaße bzw. Freiräume zwischen dem Bauelement und dem Körper mit eng tolerierten Abmessungen ermöglicht. Der an dem vorstehende Trägerchip-Bereich anliegende Körper kann einen im wesentlichen ebenen, dem Bauelement zugewandten, parallel zu dessen Oberflächenebene angeordneten und vorzugsweise an dem vorstehenden Trägerchip-Bereich anliegenden Oberflächenbereich aufweisen. Der Körper kann beispielsweise ein zweiter Trägerchip sein, der an dem vorstehenden Bereich des ersten Trägerchips plan anliegt. Die Herstellung des gegenüber dem Bauelement vorstehenden Trägerchip-Bereichs kann beispielsweise in der Weise erfolgen, daß in die Oberfläche des Trägerchips eine Vertiefung eingeätzt wird, in welcher das Bauelement angeordnet wird oder daß an bestimmten Stellen der Oberfläche des Trägerchips wenigstens eine Schicht aufgedampft oder aufgetragen wird.

Vorteilhaft ist, wenn die das elektrische oder elektronische Bauelement aufweisende Trägerchip-Oberfläche und die dieser zugewandte Oberfläche des im Bereich des Meß- oder Wirkraums des Bauelements angeordneten Vorsprungs in der Oberflächenebene der Substratplatte trichterförmig schräg zueinander verlaufen. Dadurch ergibt sich ein trichterförmiger Kanal, der für ein an der Substratplatte befindliches Medium einen strömungsrichtungsabhängigen Filter bildet.
Für eine Untersuchung oder Behandlung von biologischen Zellen ist es vorteilhaft, wenn der Abstand zwischen dem elektrischen oder elektronischen Bauelement und dem (den) im Bereich dessen (deren) Meß- oder Wirkraums angeordneten Vorsprung (Vorsprüngen) an den Durchmesser einer biologischen Zelle angepaßt ist und vorzugsweise größer als 4 µm und kleiner als 55 µm ist. Dadurch kann sich eine Zelle zwischen dem das elektrische oder elektronische Bauelement aufweisenden Überstand und dem Vorsprung unmittelbar an dem Bauelement anlagern, während Partikel, deren Abmessungen größer sind als der Zelldurchmesser von dem Bauelement ferngehalten werden.

Besonders vorteilhaft ist, wenn in die Substratplatte wenigstens zwei Trägerchips eingesetzt sind, wenn einer der Trägerchips zumindest ein als Strahlungs-Emitter ausgebildetes Bauelement und der andere Trägerchip zumindest ein als Empfänger ausgebildetes, dem Strahlungs-Emitter zugeordnetes Bauelement hat und wenn zwischen den Strahlungs-Emitter und dem Empfänger eine Meßstrecke angeordnet ist. Mit einer solchen Chip-Anordnung kann beispielsweise eine Streulicht- oder Durchlichtmessung durchgeführt werden. Dabei können die beiden Trägerchips gegebenenfalls gleichzeitig auch einen mechanischen Filter bilden, so daß nur Partikel bis zu einer bestimmten, durch den Abstand der Trägerchips vorgegebenen Größe in die Meßstrecke gelangen können.

Vorteilhaft ist, wenn die Substratplatte aus einem elastischen Material besteht. Die Substratplatte kann dann in Erstreckungsrichtung mit einer Zug- oder Druckkraft beaufschlagt werden, um den Abstand zwischen den das elektrische oder elektronische Bauelement aufweisenden Überstand des Trägerchips und einem mit diesem einen mechanischen Filter bildenden Vorsprung der Substratplatte zu verändern. Dadurch kann die Filtercharakteristik des mechanischen Filters auf einfache Weise an die Größe der zu untersuchenden oder zu behandelnden Partikel angepaßt werden. Gegebenenfalls kann die Substratplatte auch als biegbare Folie ausgebildet sein. Die Chip-Anordnung ist dann noch besser handhabbar.

Die Chip-Anordnung kann noch kostengünstiger hergestellt werden, wenn die Substratplatte wenigstens vier in einer Ebene angeordnete Plattenteile aufweist, wenn zueinander benachbarte Plattenteile jeweils an ihren einander zugewandten Randbereichen vorzugsweise durch eine Klebung miteinander verbunden sind, und wenn der Durchbruch durch einen zwischen den Plattenteilen befindlichen Freiraum gebildet ist. Dadurch kann ein teueres Bohren des Durchbruchs, beispielsweise mittels Ultraschall oder eines Laserstrahls entfallen. Auch kann an dem den Durchbruch begrenzenden Rand der Substratplatte ein Grat, wie er beispielsweise beim Laserbohren auftreten kann, vermieden werden. Die einzelnen Plattenteile weisen vorzugsweise jeweils gerade Ränder auf und können beispielsweise durch Trennschleifen oder Sägen zugeschnitten werden.

Besonders vorteilhaft ist, wenn wenigstens zwei erste Plattenteile jeweils zumindest einen geraden Randbereich aufweisen, mit denen sie parallel zueinander und einander zugewandt angeordnet sind, und wenn zwischen den ersten Plattenteilen in Erstreckungsrichtung der geraden Randbereiche durch den Durchbruch voneinander beabstandet zumindest zwei zweite Plattenteile angeordnet sind, die jeweils an ihren parallel zueinander verlaufenden Rändern mit den geraden Randbereichen der ersten Plattenteile insbesondere durch eine Klebung verbunden sind. Die aneinander anliegenden ersten und zweiten Plattenteile können dann vor dem Anbringen der Klebung in Richtung ihrer geraden Randbereiche gegeneinander verschoben werden, wodurch die Länge des in der Substratplatte befindlichen Durchbruchs auf einfache Weise verändert und an die Abmessungen des darin einzusetzenden Trägerchips angepaßt werden kann.

Zweckmäßigerweise ist der quer zur Trägerchip-Erstreckungsebene angeordnete stirnseitige Endbereich des Trägerchips zumindest im Bereich des das Bauelement aufweisenden Überstands mit einer Isolationsschicht abgedeckt. Dadurch wird bei einem Halbleiter-Trägerchip ein Kurzschluß zwischen dem Substrat des Trägerchips und einem in den Trägerchip integrierten elektronischen Bauelement, beispielsweise einem Sensor, vermieden, wenn der das elektronische Bauelement aufweisende Überstand des Trägerchips mit einem elektrisch leitfähigen Medium, zum Beispiel einem Nährmedium für biologische Zellen, in Verbindung gebracht wird.

An der den Anschlußkontakten zugewandten Rückseite der Substratplatte kann eine Leiterplatte angeordnet sein, die mit den Anschlußkontakten verbundene oder verbindbare Anschlußstellen aufweist. Dadurch ergibt sich ein besonders kompakter Aufbau. Die Leiterplatte kann beispielsweise eine Auswertevorrichtung und/oder eine Steuereinrichtung und/oder eine Stromversorgung für die Chip-Anordnung aufweisen. Diese ist an der Rückseite der Substratplatte vor Berührung mit einem zu untersuchenden Medium geschützt.

Nachfolgend sind Ausführungsbeispiele der Erfindung anhand der Zeichnung näher erläutert. Es zeigen:
- Fig. 1: einen Querschnitt durch eine Substratplatte, in die ein Trägerchip eingesetzt ist, der einen an einer Flachseite der Substratplatte vorstehenden, elektronische Bauelemente aufweisenden spitzen Überstand hat,
- Fig. 2: eine Seitenansicht einer Chip-Anordnung, bei der die Erstreckungsebenen der Substratplatte und des Trägerchips gegeneinander geneigt sind,
- Fig. 3: einen Querschnitt durch die in Fig. 2 gezeigte Chip-Anordnung,
- Fig. 4: eine Aufsicht auf die Chip-Anordnung nach Figur 1, mit einer Substratplatte, die mehrere in einer Ebene angeordnete, miteinander verklebte Plattenteile aufweist und
- Fig. 5: eine Chip-Anordnung, in deren Substratplatte zwei Trägerchips angeordnet sind, von denen eines einen optischen Sender und das andere einen diesem zugeordneten Empfänger aufweist.

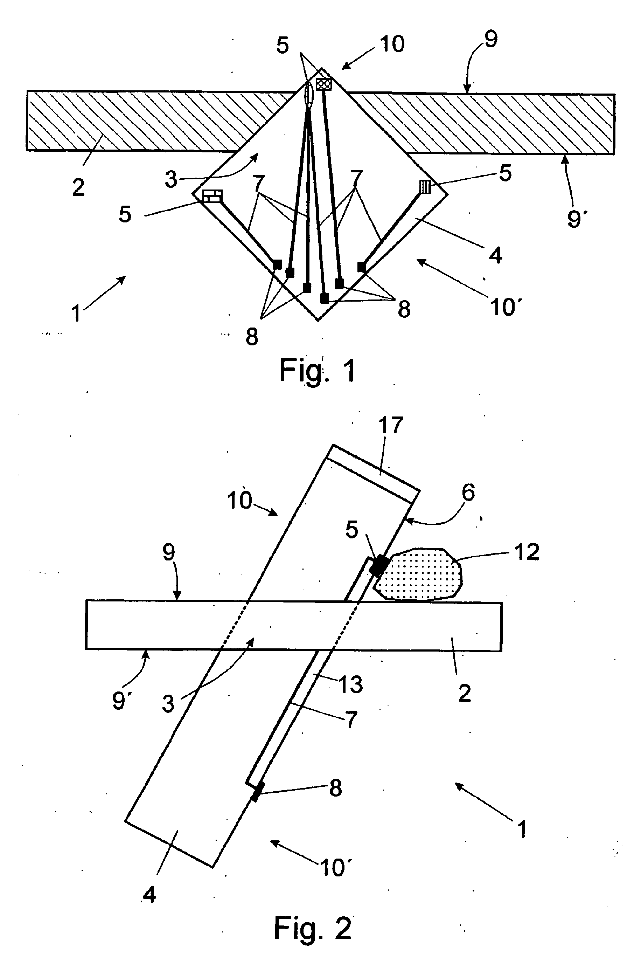

Eine im ganzen mit 1 bezeichnete Chip-Anordnung weist eine Substratplatte 2 mit einem Durchbruch 3 auf, in den ein Trägerchip 4 eingesetzt ist. Die Substratplatte 2 kann beispielsweise aus Glas oder einem Halbleitermaterial bestehen. Der Trägerchip 4 weist mehrere als Sensoren ausgebildete elektronische Bauelemente 5 auf, die an einer flachseitigen Oberfläche 6 des Trägerchips 4 mit Methoden der Halbleitertechnik in den Trägerchip 4 integriert sind. Die einzelnen Bauelemente 5 sind jeweils mit einer an der Oberfläche 6 des Trägerchips 4 oder im wesentlichen parallel dazu verlaufenden Leiterbahn 7 mit einem Anschlußkontakt 8 verbunden, an dem eine Auswerte- und Steuereinrichtung anschließbar ist. Wie aus Figur 1 besonders gut erkennbar ist, ist der Trägerchip 4 so in den Durchbruch 3 der Substratplatte 2 eingesetzt, daß er mit seinen Enden die einander abgewandten flachseitigen Oberflächen 9, 9' der Substratplatte 2 überragt und dadurch Überstände 10, 10' bildet, die an den flachseitigen Oberflächen 9, 9' der Substratplatte 2 vorstehen. Dabei sind die Bauelemente 5 an dem einen Überstand 10 und die diesen jeweils zugeordneten elektrischen Anschlußkontakte 8 an dem anderen Überstand 10' angeordnet.

Aus Figur 1 ist deutlich erkennbar, daß die die Bauelemente 5 mit den Anschlußkontakten 8 verbindenden Leiterbahnen 7 den Durchbruch 3 der Substratplatte 2 durchsetzen. Der Trägerchip 4 ist mit dem den Durchbruch 3 der Substratplatte 2 begrenzenden Rand der Substratplatte 2 verklebt, wobei der zwischen diesem Rand und dem Trägerchip 4 befindliche Klebstoff den Trägerchip 4 gegen die Substratplatte 2 abdichtet. Somit sind die an der Rückseite der Substratplatte 2 befindlichen Anschlußkontakte 8 gut gegen ein an der den Bauelementen 5 zugewandten Vorderseite der Substratplatte 2 befindliches, mit den als Sensoren ausgebildeten Bauelementen 5 zu untersuchendes Medium, das beispielsweise ein Nährmedium 11 mit darin befindlichen biologischen Zellen sein kann, abgeschirmt. Eine Korrosion an den Anschlußkontakten 8 durch in dem Nährmedium 11 enthaltene Bestandteile, wie beispielsweise Salze oder Ionen, wird dadurch zuverlässig vermieden. Da die Leiterbahnen 7 den Durchbruch 3 der Substratplatte 2 durchsetzen, brauchen bei der Herstellung der Chip-Anordnung zum Verbinden der Bauelemente 5 mit den e-elektrischen Anschlußkontakten 8 keine Durchkontaktierungen in die Substratplatte 2 eingebracht werden. Die Chip-Anordnung 1 ist dadurch einfach und kostengünstig herstellbar.

Bei dem Ausführungsbeispiel nach Figur 1 ist der Trägerchip 4 als rechteckiges Plättchen ausgebildet, das mit seiner Chipebene und seinen quer dazu verlaufenden Schmalseitenflächen jeweils rechtwinklig zu den flachseitigen Oberflächen 9, 9' der Substratplatte 2 angeordnet ist. Die den Durchbruch 3 begrenzenden Oberflächen der Substratplatte 2 sind jeweils rechtwinklig zu ihren flachseitigen Oberflächen 9, 9' angeordnet. Dadurch kann der Durchbruch 3 bei der Herstellung der Chip-Anordnung leichter in die Substratplatte 2 eingebracht werden.

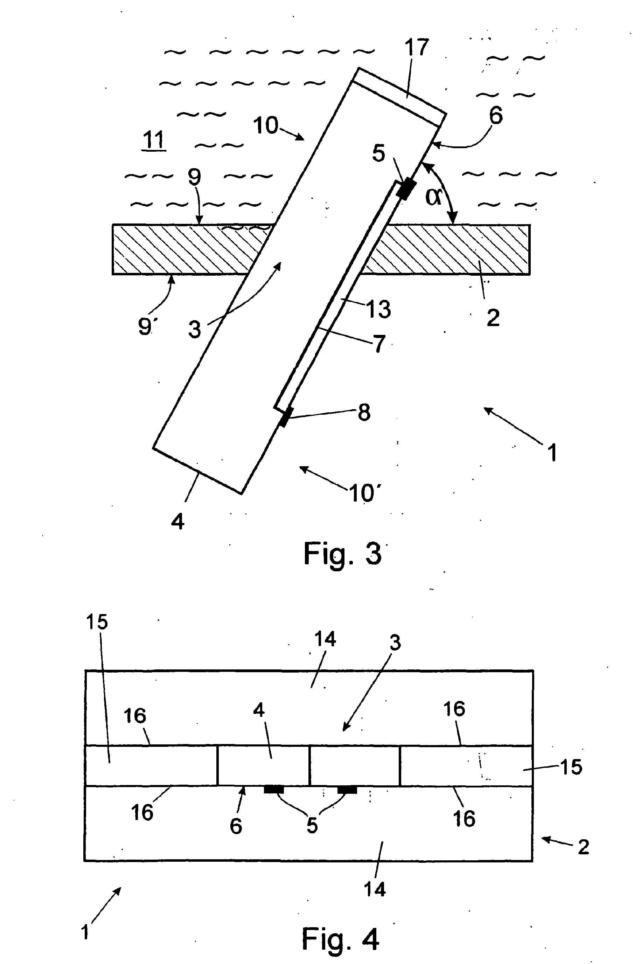

Bei dem Ausführungsbeispiel nach Figur 2 und 3 ist die das Bauelement 5 und den Anschlußkontakt 8 aufweisende Trägerchip-Oberfläche 6 in einer rechtwinklig zu den flachseitigen Oberflächen 9, 9' der Substratplatte 2 verlaufenden Ebene schräg zu diesen Oberflächen 9, 9' angeordnet und schließt mit diesen einen spitzen Winkel α ein. Das Bauelement 5 ist an der flachseitigen Oberfläche 6 des Trägerchips 4 mit Abstand zu den Rändern dieser Oberfläche 6 angeordnet. Der Zutrittsbereich zu dem Bauelement 5 ist also durch den Trägerchip 4 und die Substratplatte 2 begrenzt, wobei der Öffnungswinkel α des Zutrittsbereichs so gewählt ist, daß Partikel, die eine vorgegebene Größe überschreiten von dem Bauelement 5 ferngehalten werden.

Das Bauelement 5 kann beispielsweise ein Glucose-Sensor oder Lactat-Sensor sein. Das Bauelement 5 kann auch ein Sauerstoffsensor auf Clark-Zellenbasis, ein Stickstoffsensor, ein Sensor zur Messung einer lonenkonzentration oder ein Thermoelement sein. Es kann aber auch ein Bauelement 5 verwendet werden, das ein elektrisches oder elektromagnetisches Feld aussendet, mit dem eine an der Substratplatte 2 befindliche Zelle beeinflußt oder stimuliert werden kann.

Die Leiterbahnen 7 sind mit einer elektrisch isolierenden Dünnfilm-Passivierungsschicht 13 abgedeckt, die beispielsweise aus Siliziumoxid bestehen kann. Durch die Passivierungsschicht 13 sind die Leiterbahnen 7 gegen das Nährmedium 11 elektrisch gut isoliert. Außerdem wird durch die Passivierungsschicht 13 eine Korrosion an den Leiterbahnen 7 durch in dem Nährmedium 11 enthaltene Salze oder Ionen verhindert.

Der Querschnitt des das Bauelement 5 aufweisenden Überstandes 10 verjüngt sich ausgehend von der dem Bauelement 5 zugewandten flachseitigen Oberfläche 9 der Substratplatte zu einer an der am weitesten vorstehenden Stelle des Trägerchips 4 befindlichen spitzen Kante hin. Diese ist durch einen an der Oberfläche 9 der Substratplatte 2 vorstehenden Eckbereich des Trägerchips 4 gebildet. Wie aus Figur 1 besonders gut erkennbar ist, weist der als etwa quadratisches Plättchen ausgebildete Trägerchip 4 an diesem Eckbereich zwei in den Trägerchip 4 integrierte elektronische Bauelemente 5 auf, die mittels Leiterbahnen 7 mit Anschlußkontakten 8 verbunden sind, die an dem gegenüberliegenden, an der diesen Bauelementen 5 abgewandten flachseitigen Oberfläche 9 der Substratplatte 2 befindlichen Überstand 10' des Trägerchips 4 angeordnet sind. Der die als Sensoren ausgebildeten Bauelemente 5 aufweisende spitze Überstand 10 des Trägerchips 4 kann in einen zu untersuchenden weichen Körper, beispielsweise eine Hautschicht eingesteckt werden, wobei die flachseitige Oberfläche 9 der Substratplatte 2 als Anschlagfläche für den Körper dient, welche die Einbringtiefe des spitzen Überstands 10 in dem Körper begrenzt. Dadurch ist es möglich, die Bauelemente 5 auf einfache Weise in einer definierten, durch das Überstandmaß des Überstands 10 vorgegebenen Tiefe in dem zu untersuchenden Körper oder Medium zu positionieren.

Wie aus Figur 1 ersichtlich ist, ist der Trägerchip 4 bei der Montage der Substratplatte 2 in drei unterschiedlichen Drehlagen bezüglich der Oberflächennormalen auf die Trägerchip-Ebene in den im Querschnitt etwa trapezförmigen Durchbruch 3 der Substratplatte 2 einsetzbar, wobei in den einzelnen Drehlagen jeweils Überstände mit unterschiedlichen Bauelementen 5 an der in Gebrauchsstellung dem Meßobjekt zugewandten flachseitigen Oberfläche 9 der Substratplatte 2 angeordnet sind und diese Bauelemente 5 jeweils mittels den Durchbruch 3 durchsetzender Leiterbahnen 7 mit Anschlußkontakten 8 verbunden sind, die an den gegenüberliegenden, an der dem zu untersuchenden Meßobjekt abgewandten flachseitigen Oberfläche 9' befindlichen Überstand 10' angeordnet sind. Dadurch können je nach Wahl der Lage des Trägerchips 4 unterschiedliche Chip-Anordnungen 1 hergestellt werden. In der Chip-Ebene ist der Trägerchip mit seinen die Substratplatte 2 durchsetzenden Rändern schräg zu den flachseitigen Oberflächenebenen 9, 9' der Substratplatte 2 angeordnet. In Fig. 1 ist deutlich erkennbar, daß diese Ränder des Trägerchips 4 jeweils etwa unter einem Winkel von 45° gegenüber den Oberflächenebenen 9, 9' geneigt sind.

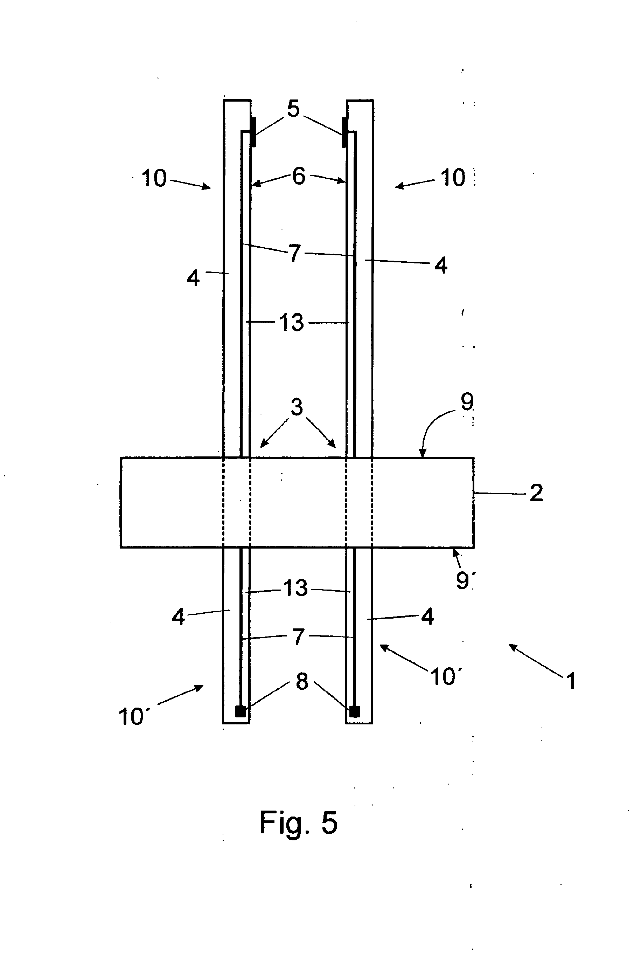

Figur 5 zeigt eine Chip-Anordnung, deren Substratplatte 2 Durchbrüche 3 für zwei einander zugeordnete Trägerchips 4 aufweist, wobei einer der Trägerchips 4 als Bauelement 5 einen optischen Sender und der andere Trägerchip 4 einen optischen Empfänger aufweist. Zwischen dem optischen Sender und dem Empfänger ist eine Meßstrecke gebildet. Die Chip-Anordnung kann beispielsweise zur Durchlicht- oder Streulichtmessung und/oder als Lichtschranke verwendet werden.

Bei dem Ausführungsbeispiel gemäß Figur 4 weist die Substratplatte 2 vier in einer Ebene angeordnete Plattenteile auf, nämlich zwei erste Plattenteile 14 und zwei zweite Plattenteile 15. Die ersten Plattenteile 14 haben jeweils einen geraden Randbereich 16, mit denen sie parallel zueinander und einander zugewandt angeordnet sind. Die zweiten Plattenteile 15 sind in Erstreckungsrichtung der geraden Randbereiche 16 nebeneinander, durch den Durchbruch 3 voneinander beabstandet zwischen den ersten Plattenteilen 14 angeordnet und jeweils an ihren parallel zueinander verlaufenden längsseitigen Rändern mit einem geraden Randbereich 16 eines ersten Plattenteils 14 verklebt. Dadurch ist es möglich, bei der Herstellung der Chip-Anordnung 1 den Trägerchip 4 entlang einer rechtwinklig zur Erstreckungsebene der Substratplatte 2 verlaufenden Geraden zu verschieben, um das Überstandsmaß bzw. die Spitzenhöhe des das Bauelement 5 aufweisenden Überstands 10 einzustellen. Dabei wird wenigstens eines der zweiten Plattenteile 15 in der Plattenebene parallel zu den geraden Randbereichen 16 der ersten Plattenteile 14 verschoben, um die Abmessungen des Durchbruchs 3 an die jeweilige Position des Trägerchips 4 anzupassen. Insgesamt ergibt sich somit ein aus den Plattenteilen 14, 15 und dem Trägerchip 4 bestehender Bausatz, mit dem Chip-Anordnungen 1, deren Überstände 10 unterschiedlich weit an der Oberfläche 9 der Substratplatte 2 vorstehen, auf einfache Weise herstellbar sind.

Bei dem Ausführungsbeispiel nach Figur 2 ist der quer zur Trägerchip-Erstreckungsebene angeordnete stirnseitige Endbereich des Trägerchips 4 im Bereich des das Bauelement 5 aufweisenden Überstands 10 mit einer Isolationsschicht 17 abgedeckt. Dadurch wird ein Stromfluß von dem elektrischen Bauelement 5 über das elektrisch leitfähige Nährmedium 11 in das Substrat der Substratplatte 2 verhindert.

## Patentansprüche

1. Chip-Anordnung (1) mit einer Substratplatte (2), die wenigstens einen Durchbruch (3) aufweist, und mit einem Trägerchip (4), der in dem Durchbruch (3) eingesetzt oder einsetzbar ist, und der an einer Trägerchip-Oberfläche wenigstens eine integrierte Leiterbahn (7) aufweist, die wenigstens ein elektrisches oder elektronisches Bauelement (5), insbesondere einen Sensor, mit zumindest einem elektrischen Anschlußkontakt (8) verbindet, wobei der Trägerchip (4) derart in den Durchbruch (3) eingesetzt oder einsetzbar ist, daß er mit seinen Enden die einander abgewandten flachseitigen Oberflächen (9, 9') der Substratplatte (2) überragt und **dadurch** Überstände (10, 10') bildet, wobei an dem die eine Oberfläche (9) überragenden Überstand (10) das Bauelement und an dem die andere Oberfläche (9') überragenden Überstand (10') der Anschlußkontakt (8) angeordnet ist, wobei die das Bauelement (5) und den Anschlußkontakt (8) miteinander verbindende Leiterbahn (7) den Durchbruch (3) der Substratplatte (2) durchsetzt, und wobei zwischen der Substratplatte (2) und dem Trägerchip (4) eine Abdichtung vorgesehen ist, **dadurch gekennzeichnet, daß** sich der Querschnitt des das elektrische oder elektronische Bauelement (5) aufweisenden Überstandes (10) ausgehend von der Oberfläche (9) der Substratplatte (2) zu der am weitesten vorstehenden Stelle des Überstandes (10) derart verjüngt, daß er in einen zu untersuchenden oder zu behandelnden weichen Körper eingesteckt werden kann.

2. Chip-Anordnung (1) nach Anspruch 1, **dadurch gekennzeichnet, daß** der Trägerchip (4) lösbar mit der Substratplatte (2) verbindbar ist.

3. Chip-Anordnung (1) nach Anspruch 1 oder 2, **dadurch gekennzeichnet, daß** die Leiterbahn(en) (7) zumindest im Bereich des das elektrische oder elektronische Bauelement (5) aufweisenden Überstandes (10) mit einer elektrisch isolierenden Dünnfilm-Passivierungsschicht abgedeckt ist (sind).

4. Chip-Anordnung (1) nach einem der Ansprüche 1 bis 3, **dadurch gekennzeichnet, daß** der Trägerchip (4) bei der Montage der Chip-Anordnung (1) in wenigstens zwei unterschiedlichen Lagen in den Durchbruch (3) der Substratplatte (2) einsetzbar ist, daß in einer dieser Lagen wenigstens ein elektrisches oder elektronisches Bauelement (5) an einem eine flachseitige Oberfläche der Substratplatte (9) überragenden Überstand (10) des Trägerchips (4) und der (die) diesem (diesen) Bauelement(en) (5) zugeordnete(n) Anschlußkontakt(e) (8) an dem die andere flachseitige Oberfläche (9') der Substratplatte überragenden Überstand (10') angeordnet ist, und daß in der anderen Lage des Trägerchips (4) das (die) Bauelement(e) (5) und der (die) Anschlußkontakt(e) (8) an demselben, eine flachseitige Oberfläche der Substratplatte (9, 9') überragenden Überstand (10, 10') des Trägerchips (4) angeordnet sind.

5. Chip-Anordnung (1) nach einem der Ansprüche 1 bis 4, **dadurch gekennzeichnet, daß** der Trägerchip (4) bei der Montage der Chip-Anordnung (1) in wenigstens zwei unterschiedlichen Lagen in den Durchbruch (3) der Substratplatte (2) einsetzbar ist, daß der Trägerchip (4) wenigstens zwei elektrische oder elektronische Bauelemente (5) aufweist, die jeweils mittels wenigstens einer Leiterbahn (7) mit zumindest einem ihnen jeweils zugeordneten elektrischen Anschlußkontakt (8) verbunden sind, und daß je nach gewählter Lage des Trägerchips (4) jeweils wenigstens eines dieser Bauelemente (5) an einem eine flachseitige Oberfläche der Substratplatte (9) überragenden Überstand (10) des Trägerchips (4) und der (die) diesem (diesen) Bauelement(en) (5) zugeordnete(n) Anschlußkontakt(e) (8) an dem die andere flachseitige Oberfläche (9') der Substratplatte überragenden Überstand (10') angeordnet ist.

6. Chip-Anordnung (1) nach einem der Ansprüche 1 bis 5, **dadurch gekennzeichnet, daß** an dem Trägerchip (4) ein Körper anliegt, der das elektrische oder elektronische Bauelement (5) überdeckt, daß als Abstandshalter an dem Trägerchip (4) mindestens ein seitlich über die Oberflächenebene des Bauelements (5) vorstehender, an dem Körper anliegender Bereich und/oder an dem Körper ein seitlich über den das Bauelement (5) überdeckenden Oberflächenbereich vorstehender, an dem Trägerchip (4) anliegender Bereich angeordnet ist, derart, daß zwischen dem Bauelement (5) und dem Körper ein den Zugang zu dem Bauelement bildender Freiraum oder Spalt angeordnet ist.

7. Chip-Anordnung (1) nach einem der Ansprüche 1 bis 6, **dadurch gekennzeichnet, daß** die Substratplatte (2) im Bereich des Meß- oder Wirkraumes des elektrischen oder elektronischen Bauelements (5) wenigstens einen Vorsprung aufweist, der zusammen mit dem das Bauelement (5) aufweisenden Überstand (10) einen mechanischen Filter bildet.

8. Chip-Anordnung (1) nach einem der Ansprüche 1 bis 7, **dadurch gekennzeichnet, daß** die das elektrische oder elektronische Bauelement (5) aufweisende Trägerchip-Oberfläche und die dieser zugewandten Oberfläche des im Bereich des Meß- oder Wirkraums des Bauelements (5) angeordneten Vorsprungs in der Oberflächenebene der Substratplatte (2) trichterförmig schräg zueinander verlaufen.

9. Chip-Anordnung (1) nach einem der Ansprüche 1 bis 8, **dadurch gekennzeichnet, daß** in die Substratplatte (2) wenigstens zwei Trägerchips (4) eingesetzt sind, daß einer der Trägerchips (4) zumindest ein als Strahlungs-Emitter ausgebildetes Bauelement (5) und der andere Trägerchip (4) zumindest ein als Empfänger ausgebildetes, dem Strahlungs-Emitter zugeordnetes Bauelement (5) hat und daß zwischen dem Strahlungs-Emitter und dem Empfänger eine Meßstrecke angeordnet ist.

10. Chip-Anordnung (1) nach einem der Ansprüche 1 bis 9, **dadurch gekennzeichnet, daß** das elektrische oder elektronische Bauelement (5) mit einer in den Trägerchip (4) integrierten Auswerte- und oder Steuereinheit verbunden ist.

## Claims

1. Chip arrangement (1) having a substrate plate (2), which has at least one perforation (3), and having a support chip (4) which has been inserted or can be inserted into the perforation (3) and which has, on a support chip surface, at least one integrated interconnect (7) which connects at least one electrical or electronic component (5), particularly a sensor, to at least one electrical connection contact (8), where the support chip (4) has been inserted or can be inserted into the perforation (3) such that its ends project above the mutually remote flat surfaces (9, 9') of the substrate plate (2), which causes it to form projections (10, 10'), where the projection (10) which projects above one surface (9) has the component arranged on it and the projection (10') which projects above the other surface (9') has the connection contact (8) arranged on it, where the interconnect (7) connecting the component (5) and the connection contact (8) to one another passes through the perforation (3) in the substrate plate (2), and where the substrate plate (2) and the support chip (4) have a seal between them, **characterized in that** the cross-section of the projection (10) which has the electrical or electronic component (5) tapers from the surface (9) of the substrate plate (2) to the furthest projecting point of the projection (10) such that it can be introduced into a soft body which is to be examined or to be treated.

2. Chip arrangement (1) according to Claim 1, **characterized in that** the support chip (4) can be detachably connected to the substrate plate (2).

3. Chip arrangement (1) according to Claim 1 or 2, **characterized in that** the interconnect(s) (7) is (are) covered by an electrically insulating thin-film passivation layer at least in the region of the projection (10) which has the electrical or electronic component (5).

4. Chip arrangement (1) according to one of Claims 1 to 3, **characterized in that** the support chip (4) can be inserted into the perforation (3) in the substrate plate (2) in at least two different positions when the chip arrangement (1) is assembled, **in that** in one of these positions at least one electrical or electronic component (5) is arranged on a projection (10) of the support chip (4) which projects above a flat surface of the substrate plate (9), and the connection contact(s) (8) associated with this (these) component(s) (5) is arranged on the projection (10') projecting above the other flat surface (9') of the substrate plate, and **in that** in the other position of the support chip (4) the component(s) (5) and the connection contact(s) (8) are arranged on the same projection (10, 10') of the support chip (4) which projects above a flat surface of the substrate plate (9, 9').

5. Chip arrangement (1) according to one of Claims 1 to 4, **characterized in that** the support chip (4) can be inserted into the perforation (3) in the substrate plate (2) in at least two different positions when the chip arrangement (1) is assembled, **in that** the support chip (4) has at least two electrical or electronic components (5) which are respectively connected by means of at least one interconnect (7) to at least one electrical connection contact (8) respectively associated with them, and **in that** depending on the chosen position of the support chip (4) at least one of these components (5) is arranged on a projection (10) of the support chip (4) which projects above a flat surface of the substrate plate (9), and the connection contact(s) (8) associated with this (these) component(s) (5) is arranged on the projection (10') projecting above the other flat surface (9') of the substrate plate, respectively.

6. Chip arrangement (1) according to one of Claims 1 to 5, **characterized in that** the support chip (4) is abutted by a body which covers the electrical or electronic component (5), **in that** as spacers the support chip (4) has at least one region arranged on it which protrudes laterally above the surface plane of the component (5) and which abuts the body, and/or the body has a region arranged on it which protrudes laterally above the surface region covering the component (5) and which abuts the support chip (4), such that the component (5) and the body have a free space or gap arranged between them which forms the access to the component.

7. Chip arrangement (1) according to one of Claims 1 to 6, **characterized in that** the substrate plate (2) has at least one protuberance in the region of the measurement or effective space of the electrical or electronic component (5), said protuberance forming a mechanical filter together with the projection (10) which has the component (5).

8. Chip arrangement (1) according to one of Claims 1 to 7, **characterized in that** the support chip surface which has the electrical or electronic component (5) and that surface of the protuberance arranged in the region of the measurement or effective space of the component (5) which faces said support chip surface run askew from one another in a funnel shape in the surface plane of the substrate plate (2).

9. Chip arrangement (1) according to one of Claims 1 to 8, **characterized in that** at least two support chips (4) have been inserted into the substrate plate (2), **in that** one of the support chips (4) has at least one component (5) in the form of a radiation emitter and the other support chip (4) has at least one component (5) which is in the form of a receiver and which is associated with the radiation emitter, and **in that** the radiation emitter and the receiver have a measurement path arranged between them.

10. Chip arrangement (1) according to one of Claims 1 to 9, **characterized in that** the electrical or electronic component (5) is connected to an evaluation and/or control unit integrated into the support chip (4).

## Revendications

1. Agencement (1) de puce électronique qui présente une plaque de substrat (2) dans laquelle au moins une perforation (3) est ménagée et une puce de support (4) insérée ou destinée à être insérée dans la perforation (3), au moins une piste conductrice (7) qui relie au moins un composant électrique ou électronique (5) et en particulier un détecteur à au moins un contact (8) de raccordement électrique étant intégré à la surface de la puce de support, la puce de support (4) étant ou pouvant être insérée dans la perforation (3) de telle sorte que ses extrémités débordent au-dessus des surfaces planes (9, 9') non tournées l'une vers l'autre de la plaque de substrat (2) et forment ainsi des saillies (10, 10'), le composant étant disposé sur la saillie (10) qui déborde de la surface (9) et le contact de raccordement (8) étant disposé sur la saillie (10') qui déborde de l'autre surface (9'), la piste conductrice (7) reliant le composant (5) et le contact de raccordement (8) en traversant la perforation (3) de la plaque de substrat (2), un joint d'étanchéité étant prévu entre la plaque de substrat (2) et la puce de support (4), **caractérisé en ce que** partant de la surface (9) de la plaque de substrat (2), la section transversale de la saillie (10) qui présente le composant électrique ou électronique (5) se rétrécit en direction de l'emplacement le plus en débord de la saillie (10) de telle sorte que la saillie puisse être enfoncée dans un corps mou à étudier ou à traiter.

2. Agencement (1) de puce électronique selon la revendication 1, **caractérisé en ce que** la puce de support (4) peut être reliée de manière libérable à la plaque de substrat (2).

3. Agencement (1) de puce électronique selon la revendication 1 ou 2, **caractérisé en ce qu'**au moins dans la zone de la saillie (10) qui présente le composant électrique ou électronique (5), la ou les pistes conductrices (7) sont recouvertes d'une couche de passivation en film mince électriquement isolant.

4. Agencement (1) de puce électronique selon l'une quelconque des revendications 1 à 3, **caractérisé en ce que** lors du montage de l'agencement (1) de puce électronique, la puce de support (4) peut être insérée en au moins deux positions différentes dans la perforation (3) ménagée dans la plaque de substrat (2), **en ce que** dans une de ces positions, au moins un composant électrique ou électronique (5) est disposé sur une saillie (10) de la puce de support (4) qui déborde de la surface plane de la plaque de substrat (9) et le ou les contacts de raccordement (8) associés à ce ou à ces composants (5) sont disposés sur la saillie (10') qui déborde de l'autre surface plane (9') de la plaque de substrat et **en ce que**, lorsque la puce de support (4) est dans l'autre position, le ou les composants (5) et le ou les contacts de raccordement (8) sont disposés respectivement sur la même saillie (10, 10') de la puce de support (4) qui déborde d'une surface plane de la plaque de substrat (9, 9').

5. Agencement (1) de puce électronique selon l'une quelconque des revendications 1 à 4, **caractérisé en ce que** lors du montage de l'agencement (1) de puce électronique, la puce de support (4) peut être insérée en au moins deux positions différentes dans la perforation (3) de la plaque de substrat (2), **en ce que** la puce de support (4) présente au moins deux composants électriques ou électroniques (5) qui sont tous reliés au moyen d'au moins d'une piste conductrice (7) à au moins un contact d'un raccordement électrique (8) respectif associé, **en ce que** selon la position que l'on a sélectionnée pour la puce de support (4), chaque fois au moins un de ces composants (5) est disposé sur une saillie (10) de la puce de support (4) qui déborde d'une surface plane de la plaque de substrat (9) et **en ce que** le ou les contacts de raccordement (8) associés à ce ou ces composants (5) sont disposés sur la saillie (10') qui déborde de l'autre surface plane (9') de la plaque de substrat.

6. Agencement (1) de puce électronique selon l'une quelconque des revendications 1 à 5, **caractérisé en ce que** sur la puce de support (4) repose un corps qui recouvre le composant électrique ou électronique (5), et **en ce qu'**au moins une zone adjacente au corps et qui déborde latéralement du plan de la surface du composant (5) et/ou une zone adjacente à la zone de la puce de support (4), au corps, et qui déborde latéralement de la partie de la surface recouvrant le composant (5) sont disposées comme écarteurs sur la puce de support (4), de manière à former un espace libre ou un interstice qui forme entre le composant (5) et le corps un accès au composant.

7. Agencement (1) de puce électronique selon l'une quelconque des revendications 1 à 6, **caractérisé en ce que** dans la zone de l'espace de mesure ou d'action du composant électrique ou électronique (5), la plaque de substrat (2) présente au moins une saillie qui forme un filtre mécanique avec la saillie (10) qui présente le composant (5).

8. Agencement (1) de puce électronique selon l'une quelconque des revendications 1 à 7, **caractérisé en ce que** la surface de la puce de support qui présente le composant électrique ou électronique (5) et la surface tournée vers celle-ci de la saillie disposée dans la zone de l'espace de mesure ou d'action du composant (5) s'étendent obliquement l'une par rapport à l'autre en forme d'entonnoir dans le plan de la surface de la plaque de substrat (2).

9. Agencement (1) de puce électronique selon l'une quelconque des revendications 1 à 8, **caractérisé en ce qu'**au moins deux puces de support (4) sont insérées dans la plaque de substrat (2), **en ce que** l'une des puces de support (4) présente au moins un composant (5) configuré comme émetteur de rayonnement, **en ce que** l'autre puce de support (4) présente au moins un composant (5) configuré comme récepteur associé à l'émetteur de rayonnement et **en ce qu'**un parcours de mesure est disposé entre l'émetteur de rayonnement et le récepteur.

10. Agencement (1) de puce électronique selon l'une quelconque des revendications 1 à 9, **caractérisé en ce que** le composant électrique ou électronique (5) est relié à une unité d'évaluation et/ou de commande intégrée dans la puce de support (4).
